# FASCICULE DE BREVET EUROPEEN

(11) **EP 1 680 105 B1**
(45) Date de publication et mention de la délivrance du brevet: **25.02.2009**
(21) Numéro de dépôt: 04805405.0
(22) Date de dépôt: 05.11.2004
(51) Int. Cl.: A61K 31/445, A61P 11/00

(54) **UTILISATION D'INHIBITEURS DE GLUCOSIDASE POUR UNE THERAPIE DE LA MUCOVISCIDOSE**
VERWENDUNG VON GLUCOSIDASE-HEMMERN ZUR BEHANDLUNG VON MUKOVISZIDOSE
USE OF GLUCOSIDASE INHIBITORS FOR THERAPY OF MUCOVISCIDOSIS

(30) Priorité: 07.11.2003 FR 0313134
(43) Date de publication de la demande: 19.07.2006
(73) Titulaire: CENTRE NATIONAL DE LA RECHERCHE SCIENTIFIQUE (CNRS), 75794 Paris Cedex 16 (FR); UNIVERSITE DE POITIERS, 86034 Poitiers Cedex (FR)
(72) Inventeur: BECQ, Frédéric, F-86280 Poitiers (FR); NOREZ, Caroline, 86000 Poitiers (FR)
(74) Mandataire: Habasque, Etienne J. Jean-François
(86) Numéro de dépôt international: PCT/FR2004/002858
(87) Numéro de publication internationale: WO 2005/046672

(56) Documents cités:
- WO-A-01/02586
- WO-A-01/02862
- WO-A-01/21769
- WO-A-98/20123
- WO-A-20/04069190
- US-A1- 2002 035 072
- ANDERSSON CHARLOTTE ET AL: "Activation of CFTR by genistein in human airway epithelial cell lines." BIOCHEMICAL AND BIOPHYSICAL RESEARCH COMMUNICATIONS, vol. 308, no. 3, 29 août 2003 (2003-08-29), pages 518-522, XP004444570 ISSN: 0006-291X
- LEE DONG-SUN ET AL: "Genistein, a soy isoflavone, is a potent alpha-glucosidase inhibitor" FEBS LETTERS, vol. 501, no. 1, 13 juillet 2001 (2001-07-13), pages 84-86, XP004257465 ISSN: 0014-5793
- ASANO N (REPRINT): "Glycosidase inhibitors: update and perspectives on practical use" GLYCOBIOLOGY, (1 OCT 2003) VOL. 13, NO. 10, PP. 93R-104R. PUBLISHER: OXFORD UNIV PRESS INC, JOURNALS DEPT, 2001 EVANS RD, CARY, NC 27513 USA. ISSN: 0959-6658., 1 octobre 2003 (2003-10-01), XP008031797
- WEI XIAOFANG ET AL: "Turnover of the cystic fibrosis transmembrane conductance regulator (CFTR): Slow degradation of wild-type and DELTA-F508 CFTR in surface membrane preparations of immortalized airway epithelial cells" JOURNAL OF CELLULAR PHYSIOLOGY, vol. 168, no. 2, 1996, pages 373-384, XP008031799 ISSN: 0021-9541

## Description

La présente invention a pour objet l'utilisation d'inhibiteurs de glucosidase spécifiés dans la revendication 1 pour la préparation de médicaments pour le traitement de la mucoviscidose.

La mucoviscidose (CF: *Cystic Fibrosis*) est la maladie génétique récessive, autosomique, létale la plus répandue dans les populations européennes et nord américaines. Le gène CF (locus 7q31) code pour la protéine trans-membranaire nommée CFTR (pour *Cystic Fibrosis Transmembrane Conductance Regulator*) (Tsui et al., 1985; Riordan et al., 1989). Des mutations du gène CF provoquent un transport anormal d'eau et d'électrolytes à travers les membranes cellulaires de divers organes comme les poumons, les glandes sudoripares, l'intestin et le pancréas exocrine. Bien qu'il existe plus de 1000 mutations de la protéine CFTR, la mutation la plus fréquente (70% des patients) est la délétion d'une phénylalanine dans le domaine NBF1 en position 508 (delF508). La principale cause de mortalité des patients CF est liée à cette délétion et conduit à des infections ou à une insuffisance pulmonaire provoqués par une augmentation de la viscosité du mucus. Cette viscosité entraîne l'occlusion des voies respiratoires et favorise les infections par les bactéries opportunistes. Une aggravation est de plus constatée au niveau digestif et notamment pancréatique (patient pancréatique-insuffisant). La protéine CFTR est une glycoprotéine de 1480 acides aminés, appartenant à la super famille des transporteurs membranaires ABC. CFTR est un canal chlorure localisé dans la membrane plasmique apicale des cellules épithéliales pulmonaires chez les individus sains. CFTR est responsable du transport transépithélial d'eau et d'électrolytes et permet chez un individu sain l'hydratation des voies aériennes pulmonaires. Chez les patients CF, cette protéine est absente des membranes plasmiques du fait d'un mauvais adressage de la protéine qui est retenue dans le réticulum endoplasmique (RE). L'hydratation des voies aériennes pulmonaires n'est plus fonctionnelle dans ce cas. La délétion delF508 perturbe le repliement du domaine NBF1 et empêche la maturation complète de la protéine qui est donc dégradée très tôt au cours de sa biosynthèse. Cependant, si la protéine delF508 atteint la membrane, elle fonctionne comme un canal chlorure. Une des clés d'un traitement de cette maladie consiste donc en un ré-adressage de delF508 vers la membrane des cellules. Une fois à la membranes, l'activité de transport de delF508 peut être stimulée par des agonistes physiologiques-endogènes ou exogènes.

Le mécanisme d'adressage de la protéine CFTR s'effectue de la manière suivante. Après sa néosynthèse, la protéine CFTR se retrouve dans la lumière du RE où elle -subit diverses glycosylations grâce à des glycosyl transférases. La protéine se retrouve avec entre autres 3 glucoses et 1 mannose N-liés. Deux des glucoses sont ôtés par les glucosidases I et II. La calnexine ou la calréticuline, chaperonnes calcium dépendantes, reconnaissent la protéine monoglucosylée et se fixent sur celle-ci via le glucose N-lié. Ces chaperonnes évitent aux différentes protéines CFTR présentes dans le RE de s'agréger entre elles et permettent la fixation d'autres chaperonnes telle que ERp57. Le complexe CFTR/Calnexine/ERp57 ainsi formé permet le repliement du CFTR. Puis, la glucosidase II enlève le glucose restant, libérant ainsi le CFTR des chaperonnes. Si le repliement est incorrect une glucosyltransférase ajoute un glucose au CFTR qui pourra à nouveau subir un ou plusieurs cycles jusqu'à ce qu'il soit bien replié. Si le repliement est toujours incorrect, une mannosidase ôte le mannose N-lié, la protéine va alors être transportée dans le cytosol, via le complexe-canal du translocon, où elle pourra être dégradée (*Ellgard & Helenius, -2003*). Ce phénomène est observé pour 80% du CFTR-WT et 99% du delF508-CFTR. Une fois dans le cytosol, la protéine est prise en charge par différentes chaperonnes telles que Hsp70, Hsp90 ou Hdj-2. Ces chaperonnes permettent à l'ubiquitine de se fixer sur le CFTR. Ainsi marqué, le CFTR est reconnu et dégradé par le complexe du protéasome 26S qui est ATP-dépendant (*Gelman et al., 2002*). Si le repliement du CFTR est considéré comme correct par la machinerie de contrôle du réticulum, la protéine pourra atteindre l'appareil de Golgi. Elle sera prise en charge par la protéine « cargo » ERGIC-53 (appartenant à la famille des lectines) qui se lie au CFTR via le mannose. La traversée du ERGIC se fait par l'intermédiaire de vésicules formées par le facteur GOP I (*Ellgard & Helenius, 2003*). Il semble que si la protéine est mal repliée, elle est sensible à l'endoglycosidase H de l'appareil de Golgi (*Cheng et al., 1990*) et sera alors ramenée dans le réticulum où elle sera dégradée. En revanche, la protéine correctement repliée, résistante à l'endoglycosidase H, sera prise en charge par le facteur VIP 36 (homologue de ERGIC-53) et amenée à la membrane apicale (*Fiedler & Simons, 1995*).

S'il a déjà été observé qu'un inhibiteur de glucosidase, la castanospermine, avait un effet sur le renouvellement de la présence de delF508 à la surface de cellules épithéliales pulmonaires (Wei et. al., 1996), il n'a en revanche jamais été décrit que ce composé était capable non seulement de restaurer l'adressage membranaire de delF508, mais également de permettre à delF508 de fonctionner en tant que transporteur ionique. A ce titre, les auteurs de cet article n'avaient formulé aucune hypothèse quant à l'utilisation possible de ce composé dans le cadre du traitement de la mucoviscidose.

Les Inventeurs ont précisément étudié ces inhibiteurs de glucosidase afin de déterminer s'ils étaient à la fois capables d'assurer l'adressage correct et spécifique de delF508 sans pour autant affecter son activité de transporteur ionique ni la viabilité cellulaire.

Le N-butyl-deoxynojirimycin (NB-DNJ), est un inhibiteur des glucosidases I et II du réticulum endoplasmique, qui a tout d'abord été développé en tant que molécule anti-virale pour l'homme. L'inhibition de ces enzymes modifie le repliement d'une glycoprotéine de l'enveloppe du virus HIV (*Human Immunodeficiency Virus*) et par conséquent, le cycle du virus s'en trouve bloqué (Platt et al., 2001). Une thérapie utilisant le NB-DNJ a été évaluée sur des patients atteints du syndrome d'immunodéficience acquise : cette molécule est bien tolérée et n'est pas cytotoxique sur des tissus en culture même à forte concentration (2mM). En outre, ces essais cliniques ont révélé que le NB-DNJ avait également un effet inhibiteur des glucosyltransférases. C'est pourquoi cette molécule, également nommée OGT 918, a été étudiée dans le traitement de la maladie du Gaucher (*Dwek et al., 2000, Cox et al., 2000*). Cette maladie génétique est due à un déficit en enzyme lysosomale, la β-glucocérébrosidase qui entraîne une accumulation de glucocérébrosides (substrat de l'enzyme). Le NB-DNJ, inhibiteur de la glucosyltransférase impliquée dans la biosynthèse des glucocérébrosides, empêche ainsi leur synthèse et leur accumulation (*Dwek et al., 2000, Cox et al., 2000*). Le NB-DNJ a obtenu l'AMM comme médicament dans la maladie du Gaucher sous le nom de Zavesca® en 2002.

WO 01/02862 décrit le traitement de troubles de l'acheminement de protéines, plus particulièrement de la mucoviscidose, en supprimant la coopération de ERp57 avec la calnexine ou la calréticuline par l'administration d'un inhibiteur de glycosylation tel que le désoxymannojirimycine (DMJ) ou le désoxynojirimycine (DNJ).

WO 01/02586 décrit une méthode de traitement des maladies génétiques, notamment de la mucoviscidose, par l'administration d'un antagoniste d'une enzyme α1,2-mannosidase tel que le 1-désoxymannojirimycine (DMJ).

US 2002/0035072 décrit l'utilisation de dérivés d'iminosucres, dans le cadre du traitement des maladies lysosomales et suggère également d'utiliser ces dérivés d'iminosucres pour le traitement de pathologies liées à des anomalies de repliement des protéines, sans citer spécifiquement la mucoviscidose.

La présente invention découle de la mise en évidence par les Inventeurs, du fait que le NB-DNJ, et autres inhibiteurs de la glucosidase en général, sont capables de restaurer l'adressage membranaire de delF508 sans altérer les autres canaux chlorure, et permettent à delF508 de fonctionner en tant que transporteur ionique.

Par l'expression « inhibiteur de glucosidase » on entend tout inhibiteur des glucosidases I et/ou II, l'inhihition de ces glucosidases pouvant être mesurée notamment selon la méthode décrite dans Platt et al., 1994.

Parmi les composés inhibiteurs de glucosidase, on peut citer ceux capables de restaurer l'adressage membranaire de delF508 sans altérer les autres canaux chlorure, et permettent à delF508 de fonctionner en tant que transporteur ionique, notamment dans le cadre d'expériences décrites ci-après effectuées sur des cellules CF15 épithéliales humaines pulmonaire homozygotes pour la délétion delF508.

Les composés inhibiteurs de glucosidase peuvent être choisis parmi les composés de formule générale (I) suivante : 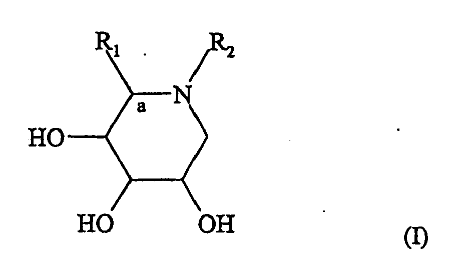 dans laquelle
- R₁ représente un groupe CH₃, ou CH₂OH
- R₂ représente H ou un groupe alkyle de 1 à 5 atomes de carbone,
- ou R₁ et R₂ forment ensemble avec la carbone en position (a) et l'azote de la formule (I) ci-dessus un groupe de formule :

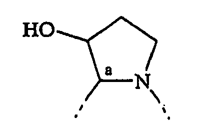

Parmi les composés de formule (I), on peut citer: 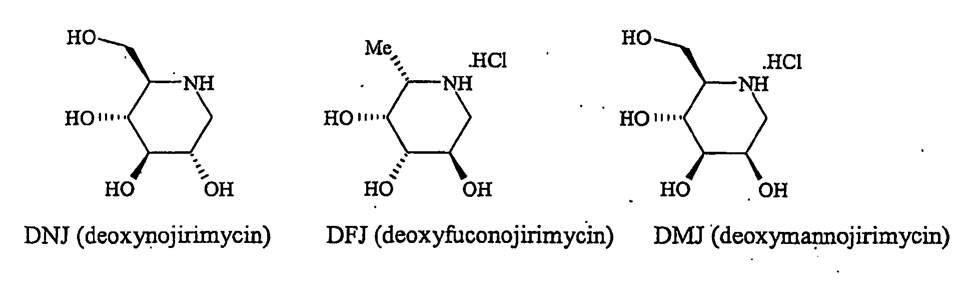 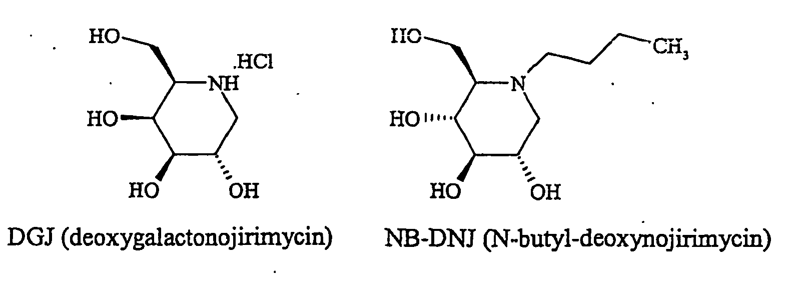 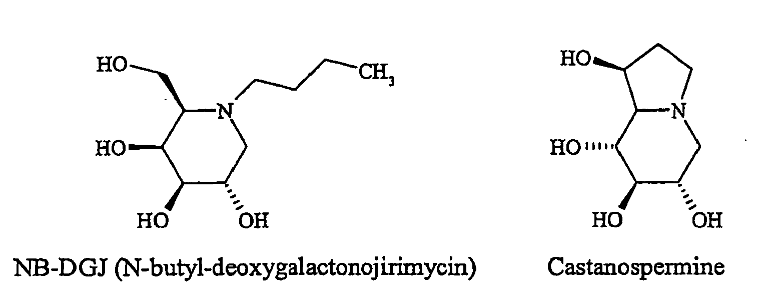 ou encore les composés de formule générale (II) suivante : 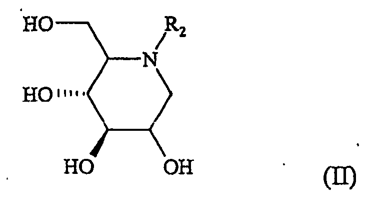 dans laquelle R₂ représente H ou un groupe alkyle de 1 à 5 atomes de carbone.

La présente invention concerne l'utilisation d'un inhibiteur de glucosidase pour la préparation d'un médicament destiné au traitement de la mucoviscidose, choisi parmi les composés de formule générale (IIbis) suivante : 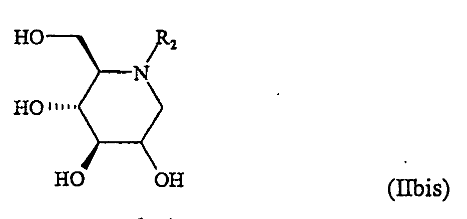 dans laquelle R₂ représente un groupe butyle.

L'invention a également pour objet l'utilisation susmentionnée d'inhibiteurs de glucosidase selon l'une des revendications 1 à 4, choisis parmi les composés de formules générales (II.1) ou (II.2) suivantes : 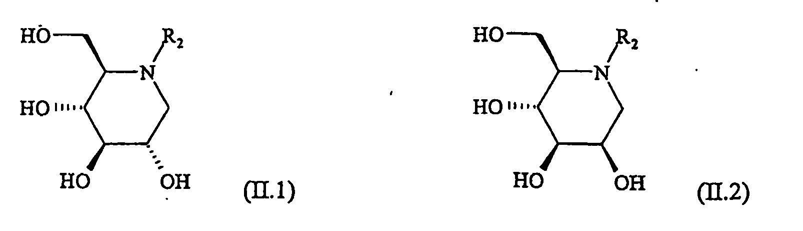 dans laquelle R₂ est tel que défini ci-dessus.

L'invention concerne plus particulièrement l'utilisation susmentionné, des composés de formules,

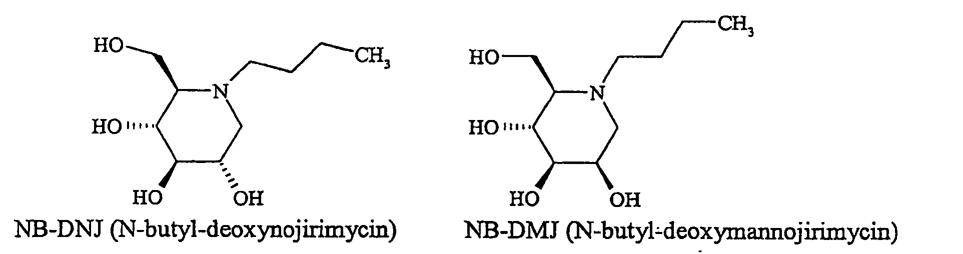

De préférence, les composés utilisés dans le cadre de la présente invention sont le NB-DNJ et le NB-DMJ.

De préférence encore, le composé utilisé dans le cadre de la présente invention est le NB-DNJ .

L'invention a également pour objet l'utilisation susmentionnée de composés inhibiteurs de glucosidase définis ci-dcssus, pour la préparation d'un médicament administrable par voies orale (sirop, suspension, gélule, tablettes, poudre ou granule), rectale (suppositoire), nasale (aérosol par inhalation, ou gouttes), notamment à raison d'environ 1 mg à 2 g par jour de composé actif chez l'adulte, ou de 1 mg à 1 g par jour chez l'enfant et le nourrisson, en une ou plusieurs prises.

Les composés inhibiteurs de glucosidase définis ci-dessus peuvent être utilisés en association avec un composé activateur du canal CFTR, choisi parmi:
- la génistéine de formule suivante : 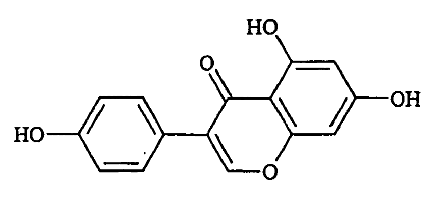
- ou les dérivés des benzo[c] quinoliziniums de formule (II) suivante : dans laquelle :

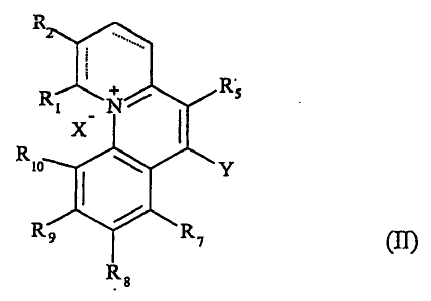

- R₁ et R₂ représentent un atome d'hydrogène, ou forment en association avec C₁ et C₂ un cycle aromatique à 6 atomes de carbone,
- R₅ représente un atome d'hydrogène, ou un groupe alkyle linéaire ou substitué de 1 à 10 atomes de carbone, notamment un groupe butyle, ou un ester de formule COOR' dans laquelle R' représente un groupe alkyle linéaire ou substitué de 1 à 10 atomes de carbone, notamment un groupe éthyle,
- Y représente un groupe -OH, -SH, -NH₂, ou -NHCOCH₃,
- R₇, R₈, R₉ et R₁₀ représentent un atome d'hydrogène, ou l'un au moins de R₇, R₈, R₉ ou R₁₀, représente un atome d'halogène, notamment un atome de chlore, de brome ou de fluor,
- X représente un atome d'halogène sous forme anionique, notamment un atome de brome Br-, ou de chlore Cl-, ou un groupe d'atomes sous forme anionique.

Les composés inhibiteurs de glucosidase définis ci-dessus peuvent être utilisés en association avec des dérivés des benzo[c] quinoliziniums de formule (II) choisis parmi les composés suivants :

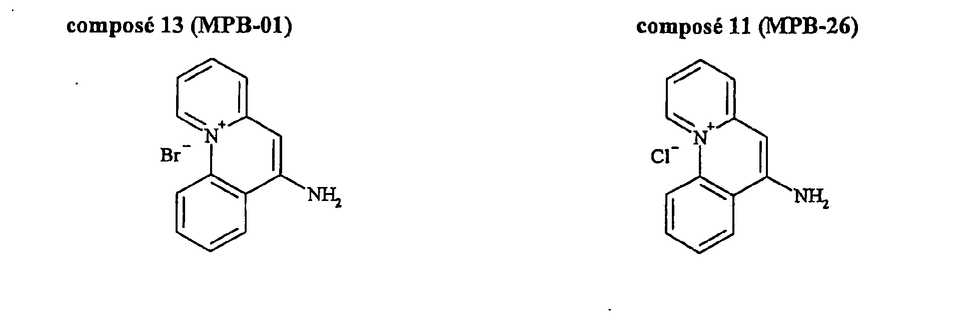

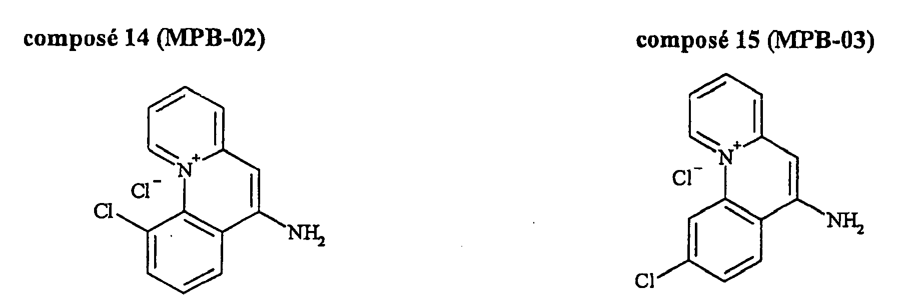

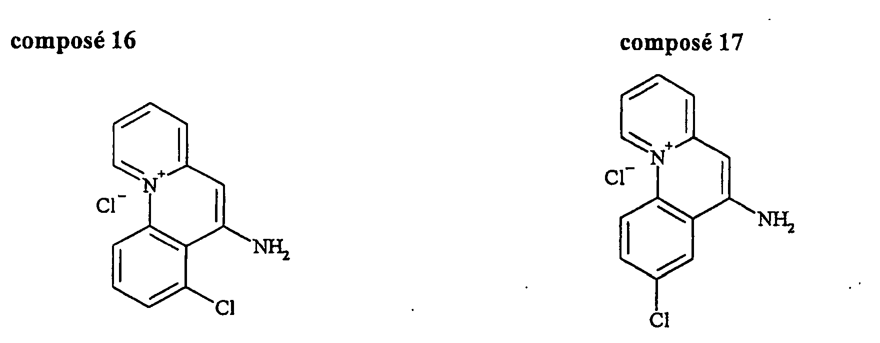

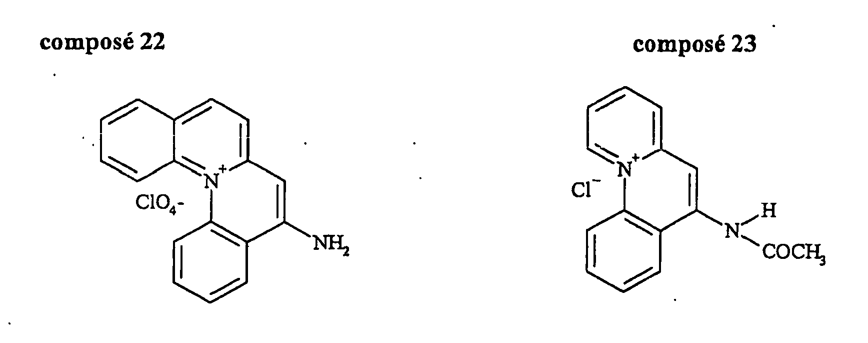

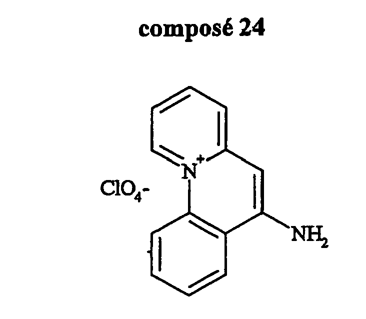

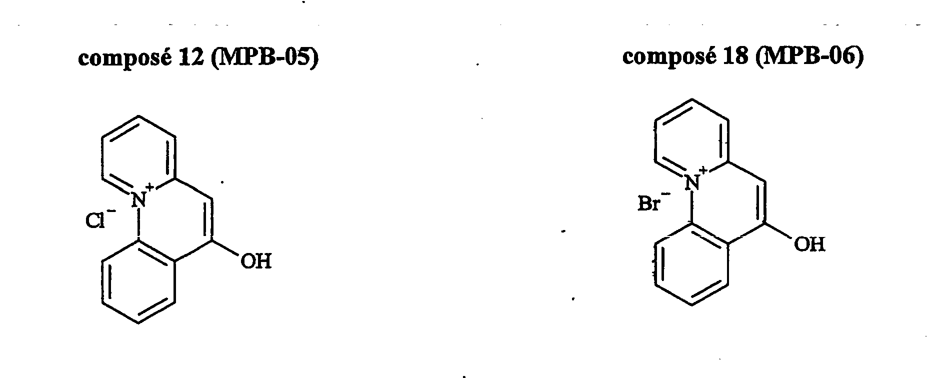

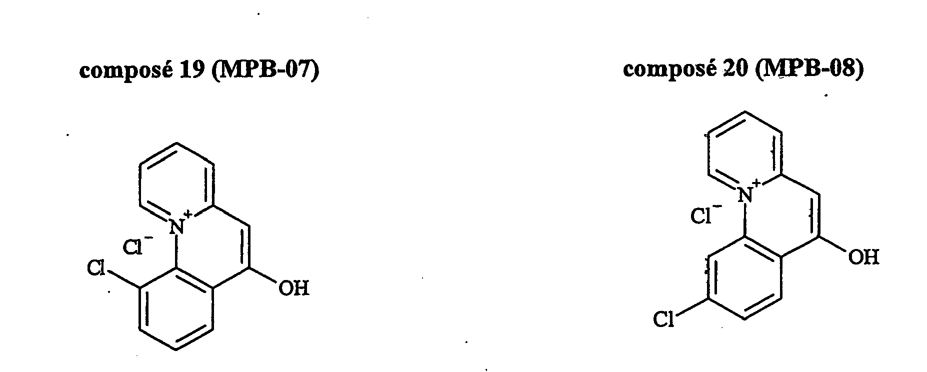

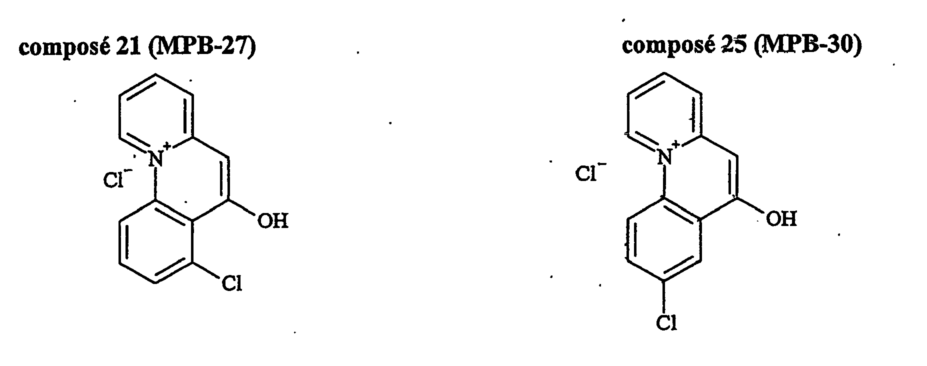

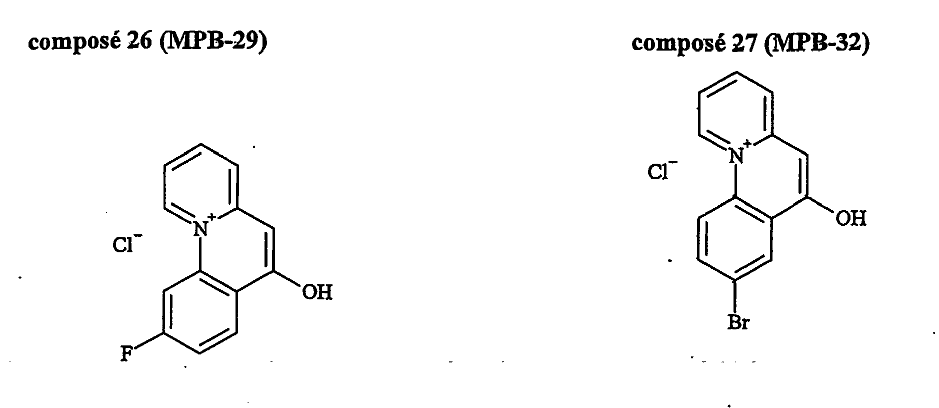

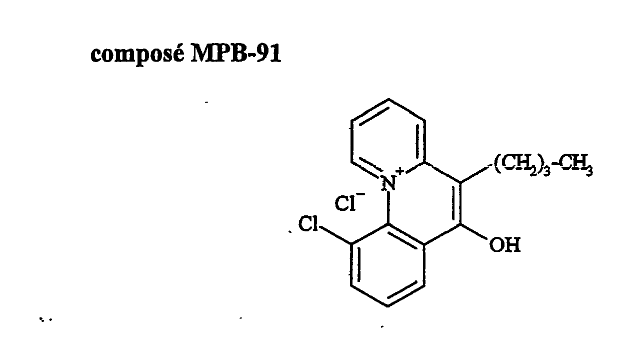

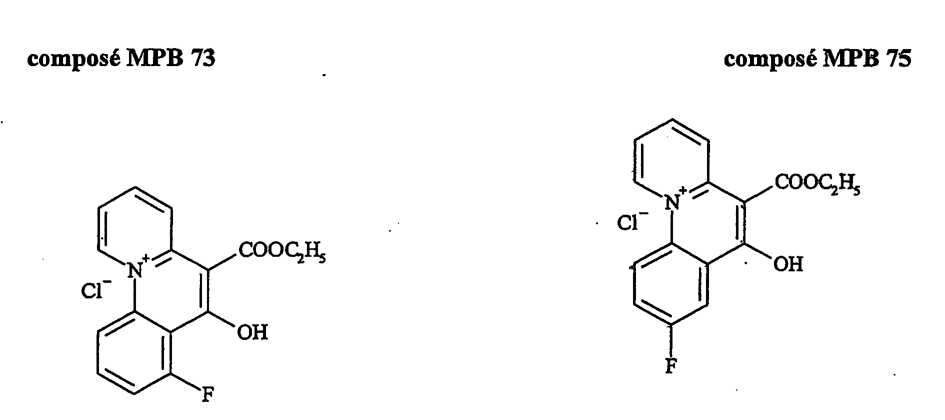

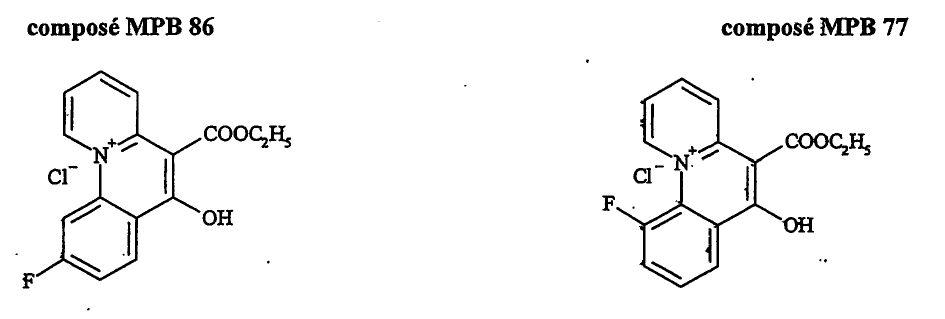

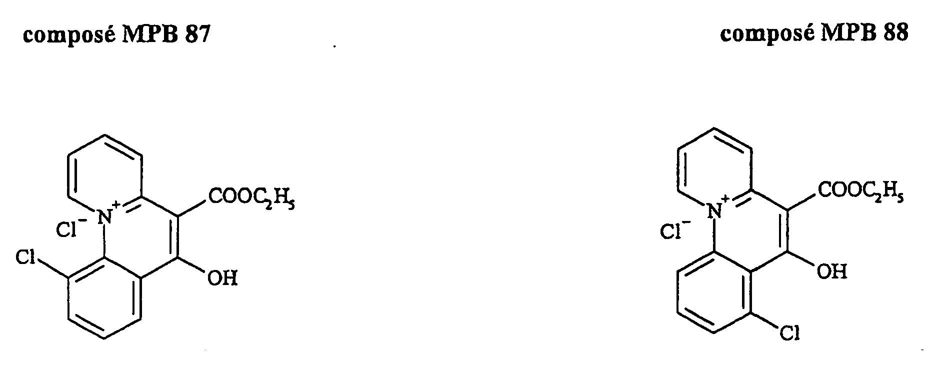

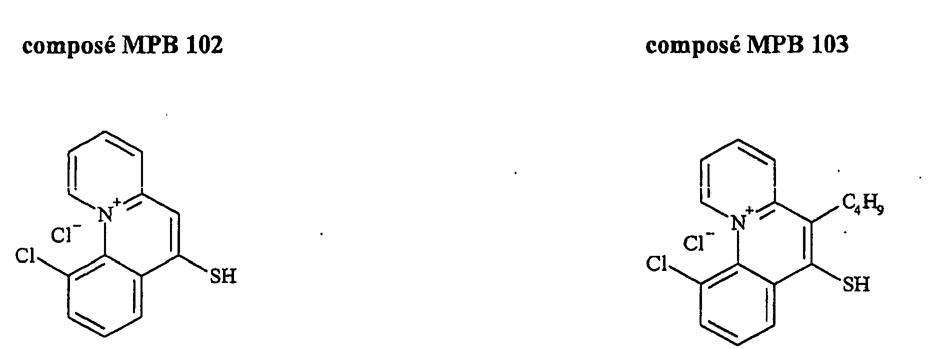

On peut également citer les produits comprenant au moins un composé inhibiteur de glucosidase, notamment un composé de formule (I) ou (II) tel que défini ci-dessus, et au moins un composé activateur du canal CFTR tel que défini ci-dessus, comme produits de combinaison pour une utilisation simultanée, séparée ou étalée dans le temps, en thérapie de la mucoviscidose.

L'invention sera davantage illustrée à l'aide de la description détaillée qui suit de la démonstration expérimentale de l'effet d'adressage correct et spécifique de delF508 par MB-DNJ, sans que ce dernier affecte pour autant son activité de transporteur ionique ni la viabilité cellulaire.

### I) MATERIEL ET METHODES

### M1. Culture cellulaire

Cellules CHO-WT : Les cellules CHO (Chinese Hamster Ovary) sont des fibroblastes qui ont été transfectées avec le gène du CFTR sauvage (CFTR-WT). Ces cellules vont donc surexprimer la protéine CFTR

Milieu de culture : Milieu MEM alpha (GIBCO) + 7% de sérum de veau foetal + 0,5% de pénicilline/streptomycine + 100 µM de Méthothrexate (Améthoptérine, Sigma)

Cellules CF15 : Les cellules CF15 sont des cellules épithéliales humaines d'origine nasale qui expriment le gène ΔF508-CFTR.

Milieu de culture: Milieu DMEM + HAM F12 + 10% de FCS + 0,6% de pénicilline/streptomycine + facteurs de croissance (insuline 5 µg/ml, transferrine 5 µg/ml, épinéphrine 5,5 µM, adénine 0,18 mM, EGF 10 ng/ml, T3 2 nM, Hydrocortisone 1,1 µM)

Cellules Calu-3 : Les cellules Calu-3 sont des cellules épithéliales humaines d'origine pulmonaire qui expriment le gène du CFTR sauvage.

Milieu de culture : Milieu DMEM/F12 avec glutamax + 7% de sérum de veau foetal + 1% de pénicilline/streptomycine

### M2. Immunomarquage

L'immunomarquage permet de visualiser la localisation cellulaire de la protéine CFTR grâce à un anticorps (Ac) primaire anti-CFTR puis un anticorps secondaire anti-anticorps primaire marqué au fluorophore Cy3. Les cellules sont préalablement ensemencées sur des lamelles dans du milieu de culture approprié. 3 lavages au TBS (NaCl : 157mM, Tris base : 20µM, pH 7,4) de 5 min chacun sont effectués. Les cellules sont alors fixées par ajout de TBS-paraformadéhyde (3%) pendant 20 min. Après 3 lavages au TBS (5 min), les cellules sont incubées avec du TBS-triton 0,1% (10 min) qui permet la formation de trous dans la membrane cellulaire, puis 3 lavages au TBS sont à nouveau effectués avant la mise en présence des cellules avec le TBS-BSA 0,5%-saponine 0,05% pendant 1 h. Les cellules sont ensuite incubées avec l'anticorps primaire anti-C terminal CFTR (2 µg/ml) pendant 1 h. 3 lavages au TBS-BSA-saponine de 5 min chacun sont réalisées avant l'incubation avec l'anticorps secondaire GAM-cy3 (1/400) pendant 1 h. Suite à 2 lavages au TBS de 5 min, les noyaux sont marqués par incubation au Topro3 (1/1000) pendant 5 min. Enfin, les lamelles peuvent être montées sur lame après 3 derniers lavages au TBS de 5 min. Les lames sont observées au microscope confocal (Bio-Rad) grâce à une excitation au laser aux longueurs d'ondes appropriées. Afin de différencier le marquage entre Cy3 et Topro3 la couleur de fluorescence de Topro3 a été changé en bleu (couleur des noyaux).

### M3. Efflux de radiotraceurs

Les mesures de transport d'ions chlorure dans les cellules ont été réalisées à l'aide de la technique des efflux d'iodure radioactif (Becq et al., 1999 ; Dormer et al., 2001). Le traceur (¹²⁵I) est incorporé dans le milieu intracellulaire. Puis, la quantité de radiotraceur qui sort de la cellule est comptée après l'ajout de différents agents pharmacologiques. L'iodure est utilisée comme un traceur du transport d'ions chlorure. Il a été montré que les deux radiotraceurs ¹²⁵I et ³⁶Cl pouvaient être considérées comme équivalents pour la mesure de l'activité d'un canal chlorure (*Venglarick et al., 1990*). ¹²⁵I a de plus l'avantage d'avoir une courte durée de vie comparée à celle de ³⁵Cl (1/2 vie respectives : 30 jours et 300000 ans). Les cellules sont mises en culture sur des plaques 24 puits dans un milieu adéquat. 2 rinçages avec du milieu d'efflux (NaCl : 136,6 mM, KCl : 5,4 mM, KH₂PO₄ : 0,3 mM, NaH₂PO4: 0,3 mM, NaHCO₃ : 4,2 mM, CaCl₂ : 1,3 mM, MgCl₂ : 0,5 mM, MgSO₄ : 0,4 mM, HEPES : 10 mM, D-glucose : 5,6 mM) sont effectués afin d'éliminer les cellules mortes qui relarguent la radioactivité de façon anarchique. Puis, les cellules sont mises à incuber avec 500 µl de charge (1 µCi/ml de ¹²⁵INa) pendant 30 min pour les CHO-WT ou 1 h pour les CF15 et Calu-3. L'iodure s'équilibre de part et d'autre de la membrane cellulaire. Le robot (MultiPROBE, Packard) réalise les étapes suivantes : Le milieu de charge est rincé avec du milieu d'efflux pour éliminer la radioactivité extracellulaire. Le surnageant est collecté toutes les minutes dans des tubes à hémolyse et le milieu est remplacé par un volume équivalent (500 µl). Les prélèvements des 3 premières minutes ne subissent pas l'addition de drogue, ils permettent d'obtenir une ligne de base stable, caractérisant la sortie passive des ions I. Les 7 prélèvements suivants sont obtenus en présence de la molécule à tester. A la fin de l'expérience, les cellules sont lysées par ajout de 500 µl de NaOH (0,1 N) / 0,1% SDS (30 min), ainsi, la radioactivité restée à l'intérieur de la cellule peut être déterminée. La radioactivité présente dans les tubes à hémolyse est comptée en coups par minute (cpm) grâce à un compteur gamma (Cobra II, Packard). Les résultats en cpm sont exprimés sous forme de vitesse de sortie d'iodure radioactif (R) d'après la formule suivante : **R (min⁻¹) = [ln(¹²⁵I t₁)-ln(¹²⁵I t₂)]/(t₁-t₂) avec ¹²⁵I t₁** : cpm au temps t₁ ; **¹²⁵I t₂**: cpm au temps t₂. Ce flux d'iodure est représenté sous forme de courbe. Afin de quantifier la sortie d'iodure due à l'administration de la molécule testée, on calcule le flux relatif suivant qui permet de s'affranchir du flux de base :**Vitesse relative (min⁻¹) = Rpic - Rbasal**. Enfin, ces résultats sont normalisés pour pouvoir comparer l'effet des différentes drogues entre elles. Les résultats sont présentés sous la forme de moyenne +/-SEM. Le test statistique de Student est utilisé pour comparer l'effet des drogues aux contrôles (les valeurs correspondantes à P<0,01 sont considérées comme statistiquement significatives).

### M4. Test de cytotoxicité

Le test de toxicité au MTT est un test colorimétrique qui repose sur la capacité des déshydrogénases mitochondriales à métaboliser le MTT (sel de tetrazolium jaune) en formazan (pourpre). L'absorbance, proportionnelle à la concentration de colorant converti, peut être alors mesurée par spectrophotométrie.Les cellules sont mises à incubées sur des plaques 96 puits en présence de l'agent à tester pendant 2 h. 3 contrôles sont réalisés: 100% cellules vivantes: cellules sans agent ; 0% cellules vivantes : cellules laissées à l'air libre ; blanc : milieu sans cellule. Les cellules sont rincées avec du milieu RPMI sans rouge de phénol pour que la couleur du milieu n'interfère pas dans les mesures de l'absorbance. Puis, elles sont incubées pendant 4 h avec 100µl de solution de RPMI supplémentée en MTT (0,5mg/ml). Le milieu est alors éliminé, l'ajout de 100 µl de DMSO permet de solubiliser le colorant converti (formazan). L'absorbance est mesurée par spectrophotométrie à :570 nm (pourpre) ; 630 nm (bruit de fond). Afin de s'affranchir du bruit de fond, le calcul suivant est effectué : **DO_{réelle} = DO₅₇₀ₙₘ-DO₆₃₀ₙₘ**. Puis, les résultats sont normalisés par rapport aux contrôles (100% et 0% de cellules vivantes) et sont présentés sous forme de moyenne +/-SEM.

### II) RESULTATS

### R1. Effet du NB-DNJ sur l'adressage de delFS08 dans les cellules CF15

L'étude de l'adressage de la protéine delF508-CFTR est réalisée au laboratoire en combinant des approches de pharmacologie, d'imagerie cellulaire, des tests biochimiques et électrophysiologiques sur des cellules CF15 épithéliales humaines pulmonaires homozygotes pour la délétion delF508. Il existe différentes molécules capables d'interférer avec certains facteurs impliqués dans l'adressage du CFTR. C'est le cas du N-butyl-deoxynojirimycin(NB-DNJ) qui est inhibiteur des glucosidases I et II et qui a été testé.

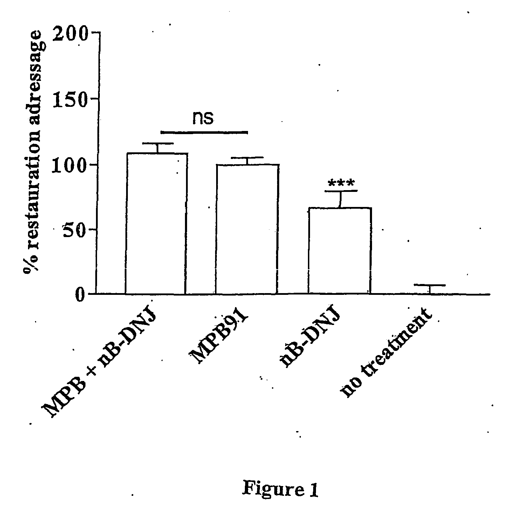

Pour chaque expérience, l'addition d'un cocktail (Forskoline 10µM, Génistéine 30µM permet l'activation du CFTR quand celui-ci est à la membrane. Ainsi, un efflux d'iodure pourra être observé si l'adressage de delF508 a été restauré. Les résultats, présentés sous forme d'histogramme ont été normalisés par rapport à un traitement de référence (traitement des cellules au MPB-91 250µM pendant 2 h) pour lequel on considère qu'on a 100% d'activité CFTR. Nous montrons ici que le traitement par un inhibiteur de glucosidase, N-butyldeoxynojirimycin (NB-DNJ) (structure présentée ci-dessous), des cellules CF15 pendant deux heures à 37°C restaure un adressage de la protéine delF508 et permet à celui ci de fonctionner en tant que transporteur ionique (figure 1).

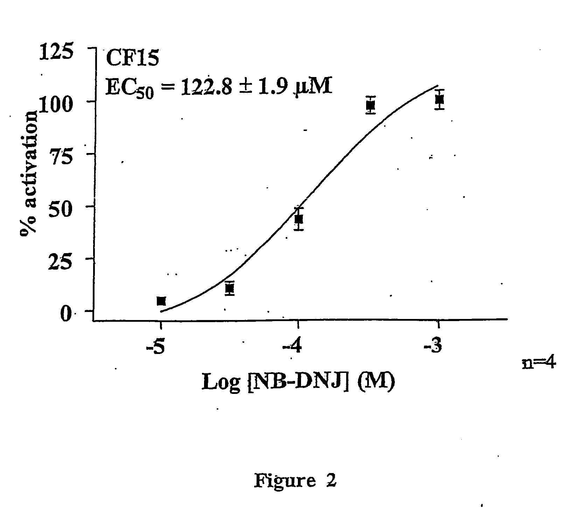

En absence de traitement des cellules, la protéine delF508 n'est pas membranaire et il n'y a pas d'efflux d'iodure stimulé par le cocktail (Forskoline 10µM, Génistéine 30µM). L'EC₅₀ (concentration de la molécule qui donné 50% de l'efficacité maximale) du NB-DNJ a été déterminé à 123 micromolaire (figure 2). Par imagerie cellulaire nous avons localisé la protéine delF508 dans les compartiments membranaires plasmatiques après traitement par le NB-DNJ.

### R2. Effet du NB-DNJ sur l'activité de CFTR dans les cellules Calu-3

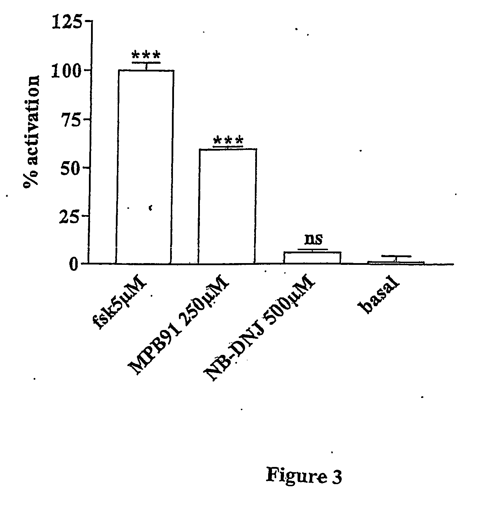

Afin de montrer que l'effet du NB-DNJ est spécifique de l'adressage du delF508 et n'altère pas les autres canaux chlorure, le NB-DNJ a été testé en tant qu'activateur potentiel sur des cellules Calu-3. Les résultats présentés dans la figure 3 ont été obtenus en efflux d'iodure sur cellules Calu-3. Nos contrôles sont la forskoline (5 µM, n=8) et le MPB-91 (250 µM, n=8). Le NB-DNJ (n=8) n'est pas un activateur du CFTR sauvage ni d'autres transports anioniques de ces cellules car il n'y a pas de différence significative entre l'effet avec ou sans NB-DNJ (basal).

### R3. Effet du NB-DNJ sur l'adressage de CFTR dans les cellules Calu-3

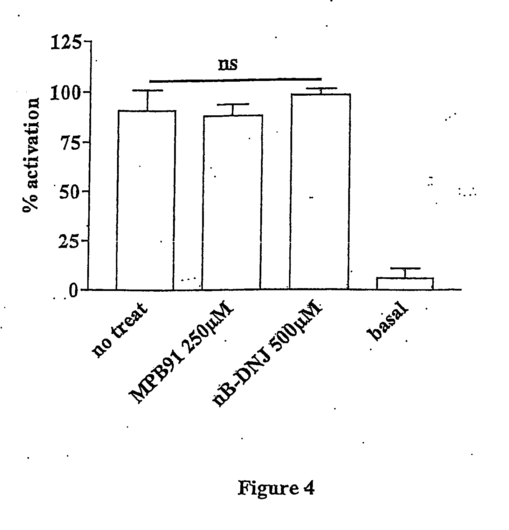

Afin de montrer que l'effet du NB-DNJ est spécifique de l'adressage du delF508 le NB-DNJ a été testé en tant que modulateur de l'adressage du CFTR sauvage sur des cellules Calu-3. Les résultats présentés dans la figure 4 ont été obtenus en efflux d'iodure sur cellules Calu-3 traitées 2 heures par du NB-DNJ (500 µM). Sur la figure 4, le basal correspond à des cellules non traitées et sans stimulation par le MPB-91. Aucun efflux d'iodure n'est stimulé. Le second contrôle correspond à des cellules sans traitement mais stimulées avec 250 µM de MPB-91. Dans ce cas CFTR est activé et un efflux d'iodure est mesuré. Le troisième contrôle correspond à des cellules traitées avec du MPB-91 2h à 37°C puis stimulées par 250µM MPB-91. L'activité CFTR dans cette condition expérimentale n'est pas différente significativement par rapport aux deux autres situations expérimentales. Enfin quand les cellules Calu-3 sont traitées 2h à 37°C par 500µM NB-DNJ et stimulées par le 250µM MPB-91, le niveau d'activité de CFTR n'est pas affecté. Ces résultats démontrent que le NB-DNJ n'affecte pas la voie d'adressage du CFTR sauvage ou d'autres canaux chlorure, ni n'altère l'activité de CFTR dans les cellules épithéliales humaines pulmonaires non-CF

### R4. Cytotoxicité des différents inhibiteurs de la voie d'adressage

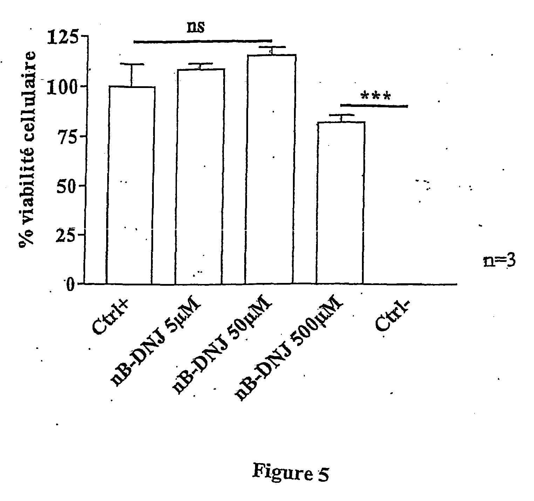

Dans le but de tester la cytotoxicité du NB-DNJ, des cellules CHO-WT ont été incubées 2h avec différentes concentrations en inhibiteurs avant de subir le test de viabilité cellulaire au MTT. La figure 5 présente un résumé des résultats. Les résultats montrent que les cellules sont viables pour toutes les concentrations de NB-DNJ. Cette molécule ne présente donc pas de cytotoxicité cellulaire.

### R5. Effet des analogues du NB-DNJ sur l'adressage de delF508 dans les cellules CF15

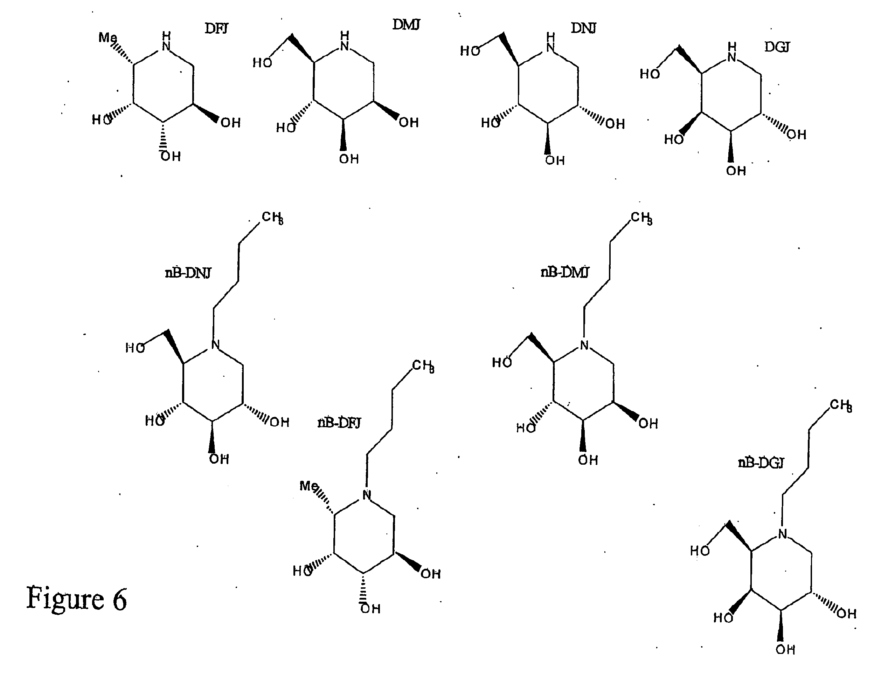

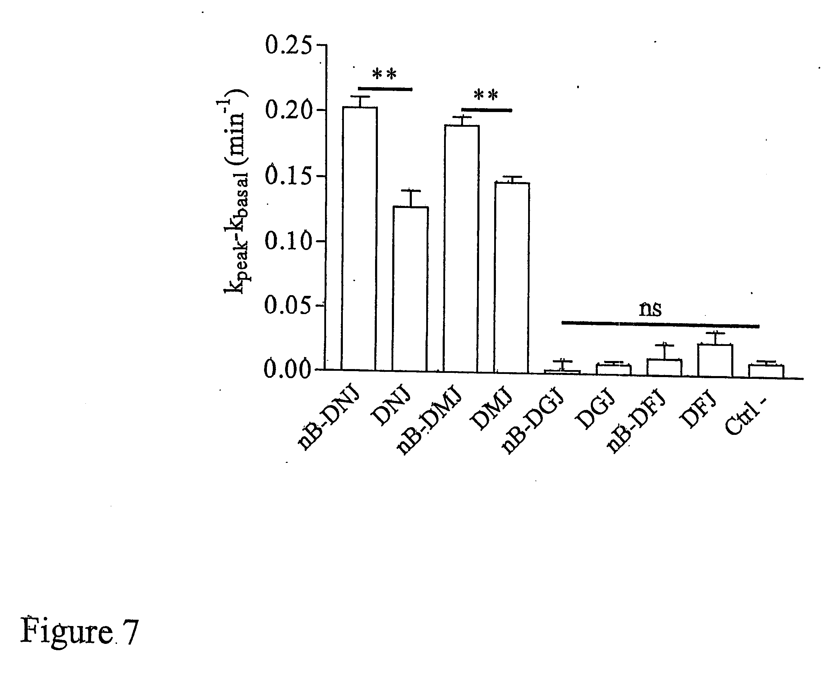

Nous avons comparé les effets de différents composés de la famille du N-butyl-deoxynojirimycin (NB-DNJ ou nB-DNJ). Les produits sont présentés dans la figure 6. Pour chaque expérience, l'addition d'un cocktail (Forskoline 10µM, Génistéine 30µM) permet l'activation du CFTR quand celui-ci est à la membrane. Les résultats présentés figure 7 montrent que le traitement des cellules CF15 par 100 µM de DNJ, nB-DMJ ou DMJ pendant deux heures à 37°C restaure un adressage de la protéine delF508 et permet à celui ci de fonctionner en tant que transporteur ionique (figure 7). Par opposition les composés DGJ, nB-DGJ, DFJ et nB-DFJ n'ont pas d'effet significatif sur l'adressage de delF508 (figure 7). Pour chaque composé (DNJ, DMJ et nB-DMJ) l'EC₅₀ (concentration de la molécule qui donne 50% de l'efficacité maximale) a été déterminé à >250 µM, 134 µM et 113µM respectivement. DFJ, DMJ, DNJ, DGJ, nB-DFJ et nB-DGJ n'entrent pas dans le cadre de la protection revendiquée.

### III) CONCLUSIONS

Les tests d'efflux ont révélé que le NB-DNJ provoquant une accumulation du delF508 dans le réticulum endoplasmiqué permet une re-localisation membranaire de cette protéine et représente donc un moyen pharmacologique important de ré-adressage du delF508 dans une cellule épithéliale humaine pulmonaire. Le NB-DNJ permet l'adressage membranaire spécifique de delF508 sans affecter l'activité de transporteur ionique de cette protéine, et n'a pas d'effet sur les autres canaux chlorure, ni sur la viabilité cellulaire.

### BIBLIOGRAPHIE

BECQ et al. (1999) Journal of Biological Chemistry 274,27415-27425.
CHENG et al. (1990) Cell 63, 827-834.
COX et al. (2000) The Lancet 355, 1481-1485.
DORMER et al. (2001) Journal of Cell Science 114, 4073-4081.
DWEK et al. (2000) Brevet WO 00/62779
ELLGAARD & HELENIUS (2003). Molecular Cell Biology, 4, 181-191.
FIEDLER & SIMONS K. (1995) Journal of Cell Science 109,271-276.
GELMAN et al. (2002) The Journal of Biological Chemistry 277, 11709-11714.
PLATT et al. (1994) The Journal of Biological Chemistry 269, 27108-27114.
PLATT et al. (2001) J. Inherit. Metab. Dis. 24, 275-290.
RIORDAN et al. (1989) Science 245, 1066-1073
TSUI et al. (1985) Science 230, 1054-1057.
WEI et al., (1996) Journal of cellular Physioly, 168, 373-384.

### LEGENDE DES FIGURES

### Figure 1 : Effet du NB-DNJ sur l'adressage de delF508.

Les cellules CF15 ont été pré-traitées 2h avec le NB-DNJ (500µM). L'effet du MPB-91 (250µM) est considéré comme représentant 100 % dans ce test. L'activité du CFTR est mesurée en efflux d'iodure (n=8 pour chaque condition) après stimulation par fsk 10µM + Gst 30µM. Ns : différence non significative.*** : différence significative P<0.001

### Figure 2 : Courbe effet du NB-DNJ sur l'adressage de delF508.

Les cellules CF15 ont été pré-traitées 2h avec le NB-DNJ à différences concentrations. L'activité du CFTR est mesurée en efflux d'iodure (n=4 pour chaque concentration) après stimulation par fsk 10µM + Gst 30µM.

### Figure 3 : Effet du NB-DNJ sur les transports anioniques des cellules Calu-3.

L'activité du CFTR est mesurée en efflux d'iodure (n=8 pour chaque condition) après stimulation par forskoline (fsk) 5µM, MPB-91 250µM,NB-DNJ 500µM. Noter que la fsk et le composé MPB-91 active un efflux d'iodure dans cette cellule, mais pas le NB-DNJ qui n'a aucun effet. Ns : différence non significative.*** : différence significative P<0.001

### Figure 4 : Effet d'un traitement au NB-DNJ des cellules Calu-3.

Les cellules Calu-3 sont pré-incubées 2 h à 37° avec 500µM NB-DNJ, avec MPB-91 250µM. Cellules non traitées : no treat. Le basal indique que les cellules n'ont ni été traitées ni stimulées. L'activité du CFTR est mesurée en efflux d'iodure (n=8 pour chaque condition) après stimulation par MPB-91 250µM. Noter qu'un traitement avec le NB-DNJ est sans aucun effet car le niveau de stimulation des afflux est le même pour les trois conditions. Ns : différence non significative.

### Figure 5 : Effet du NB-DNJ sur la cytotoxicité des cellules CHO.

Noter qu'à 5 et 50 µM aucune cytotoxicité n'est mesurable. Une faible toxicité apparaît à 500µM. 2 h de traitement des cellules. Ns : différence non significative.*** : différence significatyive P<0.001

**Figure 6** **: Structures du N-butyl-deoxynojirimycin (NB-DNJ ou nB-DNJ) et des différents composés de la famille du NB-DNJ.** DFJ, DMJ, DNJ, DGJ, nB-DFJ et nB-DGJ n'entrent pas dans le cadre de la protection revendiquée.

**Figure 7** **: Effet du NB-DNJ et des analogues du NB-DNJ sur l'adressage de delF508 dans les cellules CF15.** DNJ, DMJ, nB-DGJ, DGJ, nB-DFJ et DFJ n'entrent pas dans le cadre de la protection revendiquée.

## Revendications

1. Utilisation d'un inhibiteur de glucosidase pour la préparation d'un médicament destiné au traitement de la mucoviscidose, choisi parmi les composés de formule générale (IIbis) suivante : 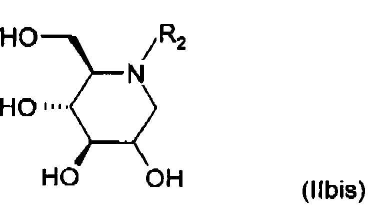 dans laquelle R₂ représente un groupe butyle.

2. Utilisation selon la revendication 1 d'un composé de formules suivantes :

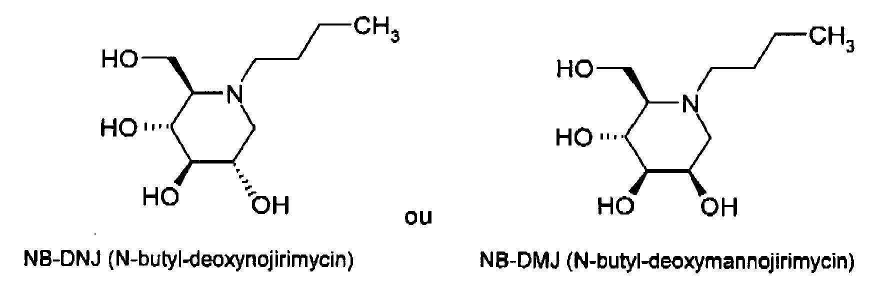

3. Utilisation selon l'une des revendications 1 ou 2 du composé NB-DNJ.

4. Utilisation de composés définis dans l'une des revendications 1 à 3, pour la préparation d'un médicament administrable par voie orale (sirop, suspension, gélule, tablettes, poudre ou granule), rectale (suppositoire), nasale (aérosol par inhalation, ou gouttes), notamment à raison d'environ 1 mg à 2g par jour de composé actif chez l'adulte, ou 1 mg à 1g par jour chez l'enfant et le nourrisson, en une ou plusieurs prises.

## Claims

1. Use of a glucosidase inhibitor for the preparation of a medicament intended for treating mucoviscidosis, which is selected from among the compounds of the following general formula (IIbis): 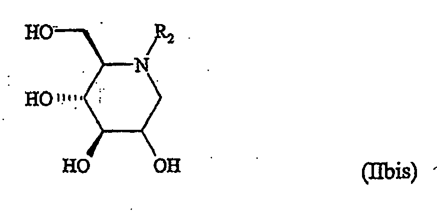 wherein R₂ denotes a butyl group.

2. Use according to claim 1 of a compound of the following formulae:

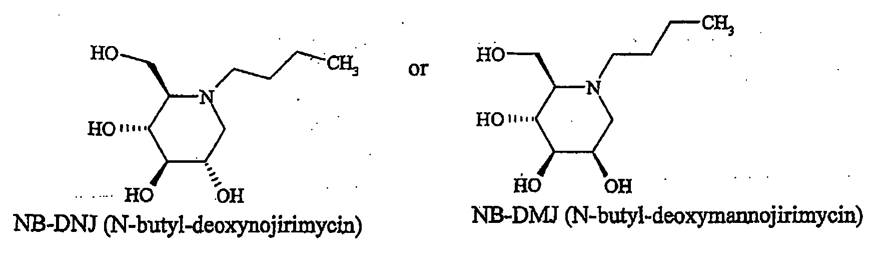

3. Use according to one of claims 1 or 2 of the compound NB-DNJ.

4. Use of compounds defined in one of claims 1 to 3, for preparing a medicament that can be administered by oral route (syrup, suspension, gelatine capsule, tablets, powder or granules), by rectal route (suppository), by nasal route (inhalable aerosol or drops), notably in an amount of about 1 mg to 2 g per day of active compound in adults, or 1 mg to 1 g per day in children and infants, in one or more doses.

## Patentansprüche

1. Verwendung eines Glukosidasehemmers zur Herstellung eines Medikaments für die Behandlung von Mukoviszidose, ausgewählt aus den Verbindungen folgender allgemeiner Formel (IIbis): 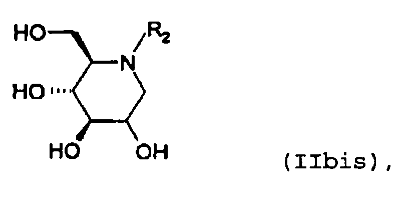 wobei R₂ eine Butylgruppe repräsentiert.

2. Verwendung nach Anspruch 1 einer Verbindung folgender Formel:

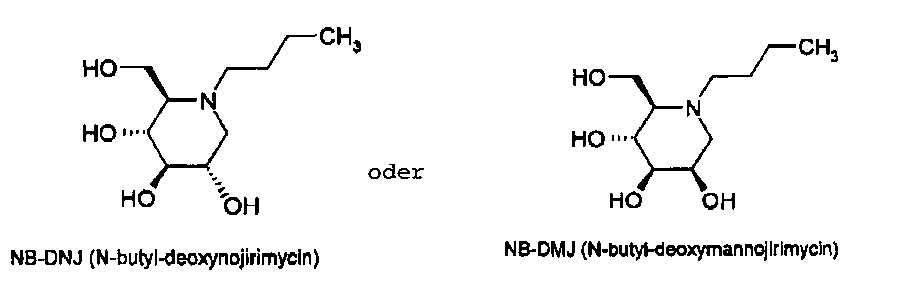

3. Verwendung nach einem der Ansprüche 1 oder 2 der Verbindung NB-DNJ.

4. verwindung bestimmter Verbindungen aus einem der Ansprüche 1 bis 3 zur Herstellung eines Medikaments, wobei das Medikament oral (Sirup, Suspension, Kapsel, Tabletten, Puder oder Granulat), rektal (Zäpfchen), nasal (Inhalationsspray oder Tropfen), insbesondere in Höhe von ungefähr 1 mg bis 2 g der wirksamen Verbindung pro Tag bei einem Erwachsenen, oder 1 mg bis 1 g pro Tag bei einem Kind oder einem Säugling, in einer oder mehreren Gaben verabreichbar ist.
